# EUROPEAN PATENT SPECIFICATION

(11) **EP 4 275 786 B1**
(45) Date of publication and mention of the grant of the patent: **18.02.2026**
(21) Application number: 22172282.0
(22) Date of filing: 09.05.2022
(51) Int. Cl.: B01J 19/00, B01F 33/30, B01L 3/00, C07C 229/22

(54) **FULL CONTINUOUS-FLOW PREPARATION METHOD OF L-CARNITINE**
VERFAHREN ZUR HERSTELLUNG VON L-CARNITIN IN EINEM VOLLSTÄNDIG KONTINUIERLICHEN VERFAHREN
PROCÉDÉ DE PRÉPARATION DE LA L-CARNITINE EN FLUX CONTINU INTÉGRAL

(43) Date of publication of application: 15.11.2023
(73) Proprietor: Fudan University, Shanghai 200433 (CN)
(72) Inventor: CHEN, Fener, Shanghai, 200433 (CN); LIU, Minjie, Shanghai, 200433 (CN); JIANG, Meifen, Shanghai, 200433 (CN); CHENG, Dang, Shanghai, 200433 (CN); YU, Chao, Shanghai, 200433 (CN); HUANG, Huashan, Shanghai, 200433 (CN)
(74) Representative: Vitina, Maruta

(56) References cited:
- EP-A2- 0 210 672
- US-A1- 2021 380 524
- US-A1- 2022 056 489

## Description

### TECHNICAL FIELD

This application relates to organic chemical engineering, and more particularly to a full continuous-flow preparation method of L-carnitine.

### BACKGROUND

L-carnitine is an important naturally-occurring substance shown in formula (1), and has a brilliant application prospect in the preparation of medicines, food additives and feed additives.

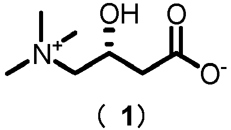

At present, the L-carnitine is prepared mainly by extraction, biosynthesis and chemical synthesis. Originally, the L-carnitine is extracted from the beef extract, but this approach has numerous and complicated operations and low yield, and thus is not suitable for the large-scale production. The biosynthesis method is mainly performed by converting crotonbetaine into L-carnitine via microbial fermentation or enzymatic catalysis. Unfortunately, the existing biosynthesis strategies still suffer poor conversion rate and troublesome isolation and purification. The chemical synthesis mainly includes two routes. In the first route, the L-carnitine is obtained in the presence of an expensive resolving agent, and this process involves a waste of D-carnitine and low yield (Chinese patent application No. 200410068172.9). With regard to the second synthetic route, the chiral epichlorohydrin or ethyl 4-chloroacetoacetate is used as raw material for synthesis, which requires a highly toxic cyanide or an expensive chiral catalyst (Chinese patent application Nos. 201210089733.8 and 201010198458.4). US patent application publication No. 2021380524 A1 discloses a method for preparing L-carnitine from an intermediate (R)-4-halo-3-hydroxybutyrate using a micro-reaction system, where the micro-reaction system includes a micro-mixer and a micro-channel reactor in communication, and the method includes: (1) pumping a (R)-4-halo-3-hydroxybutyrate and an aqueous trimethylamine solution containing an inorganic base into the micro-mixer simultaneously followed by mixing to obtain a mixture; and (2) allowing the mixture flowing out of the first micro-mixer to enter the micro-channel reactor; and subjecting the mixture to continuous quaternization and hydrolysis to obtain the L-carnitine. EP patent application publication No. 0210672 A2 discloses a process for the preparation of β -hydroxy- γ -trimethylamino-butyric acid (L-carnitine), including: reacting diketene with chlorine; submitting the obtained chlorination product to amidation by the methylester of an optically-active aminoacid to yield 4-chloro-3-ketobutyrylamide of the methylester of an optically-active aminoacid (compound IV); reducing the compound IV in the presence of a reducing agent; separating and recovering (3R)-4-chloro-3-hydroxybutyrylamide of the methylester of an optically-active aminoacid (compound VI); submitting the compound VI to methanolysisby hydrogen chloride gas in methanol; recovering the compound methylester of (3R)-4-chloro-3-hydroxybutyric acid (compound VIII); and treating the compound VIII with trimethylamine followed by hydrolysis with hydrochloric acid to obtain the L-carnitine, where the methanolysis is carried out in methanol saturated with hydrogen chloride gas under boiling conditions for 24 h. This method enables the *de novo* synthesis of L-carnitine, but it struggles with complicated operation, harsh reaction conditions and large energy and time consumption.

Besides, the traditional batch reactor-based synthesis methods also struggle with large time consumption, complicated operation, serious safety hazard, low efficiency and high energy consumption, and thus are not suitable for the large-scale production.

### SUMMARY

An objective of this application is to provide a full continuous-flow preparation method of L-carnitine to overcome the defects of large time consumption, serious safety hazard, high energy consumption and low efficiency in the prior art. The preparation method provided herein has shortened reaction time, optimized reaction efficiency, high degree of automation, improved safety and reduced energy consumption, and thus is suitable for the industrial application.

Technical solutions of this application are described as follows.

This application provides a full continuous-flow preparation method of L-carnitine using a micro-reaction system, wherein the micro-reaction system comprises a first micromixer, a first microchannel reactor, a second micromixer, a second microchannel reactor, a third micromixer, a third microchannel reactor, a fourth micromixer and a fourth microchannel reactor communicated in sequence; and the method comprises:
(S1) respectively transporting chlorine gas and a diketene reaction liquid to the first micromixer for mixing, and allowing the reaction mixture in the first micromixer to flow into the first microchannel reactor followed by continuous chlorination reaction;
(S2) transporting the reaction mixture flowing out from the first microchannel reactor and an alcohol solvent into the second micromixer and the second microchannel reactor in sequence for continuous esterification reaction;
(S3) neutralizing the reaction mixture flowing out from the second microchannel reactor with a first alkali followed by continuous extraction and separation to collect a first organic phase; simultaneously transporting the first organic phase and an aqueous solution of a reductase to a third micromixer and a third microchannel reactor for continuous reduction reaction;
(S4) removing the reductase from the reaction mixture flowing out from the third microchannel reactor via an extraction separator followed by continuous extraction and separation to obtain a second organic phase; and concentrating the second organic phase to obtain a concentrated liquid;
(S5) simultaneously transporting the concentrated liquid obtained in step (S4) and a trimethylamine solution to a fourth micromixer and a fourth microchannel reactor in sequence for continuous substitution and hydrolysis reaction; and
(S6) neutralizing the reaction mixture flowing out from the fourth microchannel reactor with a second alkali followed by feeding into a demineralization device and a concentration device to obtain the L-carnitine;
as shown in the following reaction scheme: 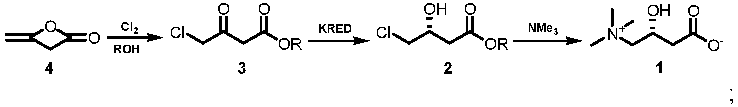
wherein R is C₁-C₆ alkyl, C₁-C₆ cycloalkyl or benzyl.

In an embodiment, the diketene reaction liquid is a solution of diketene in an organic solvent; the organic solvent is selected from the group consisting of benzene, methylbenzene, an ester solvent, a chlorinated hydrocarbon solvent and an alkane solvent, preferably methylbenzene or dichloromethane; and the alcohol solvent is selected from the group consisting of C₁-C₆ alkyl alcohol, C₁-C₆ cycloalkyl alcohol and benzyl alcohol, preferably methanol or ethanol;
the reductase is a carbonyl reductase. In this embodiment, the reductase is a carbonyl reductase genetically-engineered whole cell; and
the trimethylamine solution is a trimethylamine aqueous solution or a trimethylamine alcoholic solution, preferably a trimethylamine aqueous solution.

In an embodiment, in step (S1), the first microchannel reactor is a tubular microchannel reactor, a plate-type microchannel reactor, or other commercially available microchannel reactors.

In an embodiment, in step (S1), a flow ratio of the chlorine gas to the diketene reaction liquid is controlled such that a molar ratio of diketene to the chlorine gas is 1:(0.9~1.5), preferably 1:(1.0~1.2); the first micromixer and the first microchannel reactor are both controlled at -20~50°C, preferably 0~20°C; a residence time of the reaction mixture in the first microchannel reactor is 0.1-30 min, preferably 0.5-1 min; and a back pressure during the continuous chlorination reaction is controlled to 0.1~1.0 MPa, preferably 0.2~0.5 MPa.

In an embodiment, in step (S2), the alcohol solvent is ethanol; a flow ratio of the reaction mixture obtained in step (S1) to the ethanol is controlled such that a molar ratio of diketene to ethanol is 1:(0.9~1.5), preferably 1:(1.0~1.2); the second micromixer and the second microchannel reactor are both controlled at 20-80°C, preferably 30-50°C; a residence time of the reaction mixture in the second microchannel reactor is 0.1-30 min, preferably 5-10 min; and a back pressure during the continuous esterification reaction is controlled to 0.1-1.0 MPa, preferably 0.2-0.5 MPa.

In an embodiment, in step (S3), the third micromixer and the third microchannel reactor are both controlled at 20-50°C, preferably 30-40°C; a residence time of the reaction mixture in the third microchannel reactor is 1-30 min, preferably 10-15 min, and a back pressure during the continuous reduction reaction is controlled to 0.1-1.0 MPa, preferably 0.2-0.5 MPa.

In an embodiment, in step (S5), a flow ratio of the concentrated liquid obtained in step (S4) to the trimethylamine solution is controlled such that a molar ratio of diketene to trimethylamine is 1:(1.0~2.0), preferably 1:(1.0~1.5); the fourth micromixer and the fourth microchannel reactor are both controlled at 20-50°C, preferably 30-40°C; a residence time of the reaction mixture in the fourth microchannel reactor is 1-30 min, preferably 10-15 min; and a back pressure during the continuous substitution and hydrolysis reaction is 0.1-1.0 MPa, preferably 0.2-0.3 MPa.

In an embodiment, the first micromixer, the second micromixer, the third micromixer and the fourth micromixer are each independently selected from the group consisting of static mixer, T-type micromixer, Y-type micromixer, cross-type mixer, coaxial flow micromixer and flow-focusing micromixer, preferably the cross-type mixer, coaxial flow micromixer or flow-focusing micromixer.

In an embodiment, the first microchannel reactor, the second microchannel reactor, the third microchannel reactor and the fourth microchannel reactor are each independently selected from the group consisting of tubular microchannel reactor, plate-type microchannel reactor, and other commercially available microchannel reactors.

In an embodiment, an inner diameter of the tubular microchannel reactor is 50 µm-10 mm, preferably 100 µm-5 mm; the plate-type microchannel reactor comprises a first heat exchange layer, a reaction layer and a second heat exchange layer arranged in sequence from top to bottom; the reaction layer is provided with a reaction fluid channel; and a hydraulic diameter of the reaction fluid channel is 50 µm-10 mm, preferably 100 µm-5 mm.

In an embodiment, the continuous extraction separator is a plate-type microchannel extraction separator, a membrane extraction separator, an annular centrifugal extraction separator, a ceramic membrane separator, an electrodialysis device, a continuous concentration device, or a multi-effect falling-film evaporator.

In an embodiment, the plate-type microchannel extraction separator has an inner diameter of 100 µm-10 mm, and comprises a mixing layer and a separation layer; the membrane extraction separator has a hydrophobic membrane with a pore size of 0.1-4 µm; and the annular centrifugal extraction separator has a diameter of 10 cm-1 m, and one or more annular centrifugal extraction separators are connected in series to form an extraction-separation unit.

The full continuous-flow preparation method of L-carnitine using a micro-reaction system provided herein can realize the industrial large-scale production of L-carnitine through a multi-channel parallel amplification strategy.

Compared with the prior art, this application has the following beneficial effects.

In the method provided herein for preparing L-carnitine, a full continuous-flow micro-reaction system including a micromixer, a microchannel reactor and a continuous extraction separator communicated in sequence is adopted. Compared with the traditional batch reactor-based synthesis, this application has the following beneficial effects.
1. The full continuous-flow microchannel reaction system has excellent mass transfer, heat transfer and mixing performances, significantly shortening the reaction time from several days (required by the traditional batch reactor) to about 30 min and improving the reaction efficiency. Moreover, the L-carnitine prepared thereby has superior quality and property.
2. By means of the micro-reaction system, the consumption of chlorine gas can be precisely controlled to allow the complete and quantitative conversion of chlorine gas in the microchannel reactor and the safe operation, and prevent the system from being corroded by the excessive gas.
3. The preparation method provided herein achieves the continuous synthesis of L-carnitine in the absence of external intervention, and has high degree of automation, great time and space efficiency, reduced labor intensity and lowered production costs.
4. The preparation method provided herein can be applied to the industrial production through the multi-channel parallel amplification strategy.

### DETAILED DESCRIPTION OF EMBODIMENTS

In order to clearly explain the technical solutions, structural features, objectives and advantages of the technical solutions, the disclosure will be described in detail below with reference to the embodiments and accompanying drawings. It should be understood that the embodiments are merely illustrative, and are not intended to limit the scope of the disclosure.

This application will be described in detail below with reference to the embodiments to make the objectives, technical solutions, and advantages of this application clearer.

### Example 1

Chlorine gas and a solution of diketene in dichloromethane were simultaneously fed to a Protrix microchannel reactor (Chemtrix BV, Protrix) (reaction volume: 4.2 mL; and microchannel diameter: 2 mm), where a flow ratio of the diketene solution to chlorine gas was adjusted to allow a molar ratio of diketene to chlorine to be 1: 1.15; a back pressure value of a back pressure valve was set to 0.2 MPa; the Protrix microchannel reactor was controlled at 10°C. 30 seconds later (namely, a residence time of a reaction mixture in the Protrix microchannel reactor was 30 seconds), the reaction mixture was allowed to flow out from an outlet of the Protrix microchannel reactor to enter a gas-liquid separator, so as to remove gas components. The remaining reaction mixture was collected to a buffer tank, and sampled for detecting the conversion rate of diketene. A 100% conversion of diketene was confirmed. Then the reaction mixture in the buffer tank and ethanol were fed to a first T-type micromixer for mixing, where flow rates of the two feeding pumps were adjusted to allow a molar ratio of diketene to ethanol to be 1:1.05; and the first T-type micromixer was controlled at 25°C. The reaction mixture was subsequently transported to a tubular microchannel reactor (10 m in length and 0.8 mm in inner diameter), where a back pressure value of the back pressure valve was set to 0.3 MPa; and the first tubular microchannel reactor was controlled at 50°C. After reacted for 5 minutes (namely, a residence time of the reaction mixture in the first tubular microchannel reactor was 5 minutes), the reaction mixture was allowed to flow out from an outlet of the first tubular microchannel reactor and transported with a saturated sodium bicarbonate solution to a second T-type micromixer with a temperature controlled at 25°C. After that, the reaction mixture was continuously transported to a first continuous extraction separator, and at the same time, a dichloromethane solvent was fed as an extractant from another inlet into the first continuous extraction separator with a temperature therein controlled at 25°C. After 1 min (namely, a residence time of the reaction mixture in the first continuous extraction separator was 1 min), an organic phase containing a compound (3) was collected from a heavy phase outlet of the first continuous extraction separator, and an aqueous phase was discharged from a light phase outlet.

The obtained organic phase and a reductase aqueous solution were delivered to a third T-type micromixer for mixing, and the reaction mixture was fed to a plurality of series-connected second tubular microchannel reactors (2 m in length of a single tube and inner diameter in 0.8 mm) with pH adjustment and reacted for 15 min (namely, a residence time of the reaction mixture in the tubular microchannels was 15 min), where the back pressure value of the back pressure valve was set to 0.2 MPa, and the second tubular microchannel reactors were controlled at 30°C. The reaction mixture was sampled for detecting a conversion rate of the compound (3), and the results demonstrates the complete conversion of the compound (3). The reaction mixture was allowed to flow out from an outlet of the microchannel reactors, pass through a ceramic membrane separator to remove the reductase and enter a second continuous extraction separator, where an aqueous phase was discarded, and an organic phase was collected and treated via a continuous concentration device to remove dichloromethane, so as to obtain a compound (2).

The obtained compound (2) and a trimethylamine aqueous solution were fed to a fourth T-type micromixer for mixing, and then sent to a third tubular microchannel reactor (10 m in length and 0.8 mm in inner diameter), where flow rates of the two feeding pumps were adjusted such that a molar ratio of diketene to trimethylamine was 1:1.3; a back pressure value of the back pressure valve was set to 0.3 MPa, and the third tubular microchannel reactor was controlled at 25°C. After reacted for 10 min (namely, a residence time of the reaction mixture in the third tubular microchannel reactor was 10 min), the reaction mixture was sampled for detecting a conversion rate of the compound (2). The results confirmed that the compound (2) experienced a complete conversion. The reaction mixture was allowed to flow out from an outlet of the third tubular microchannel reactor, and fed to a fifth T-type micromixer with a dilute hydrochloric acid solution, where the fifth T-type micromixer was set to 25°C. Then the reaction mixture was transported to an electrodialysis device for desalination, and a multi-effect falling-film evaporator for concentration, and subjected to crystallization to obtain a white solid L-carnitine with a purity of 99% (65% yield calculated by diketene).

### Example 2

**Example 2** was basically the same as the **Example 1,** except that the micromixer used herein was a Y-type micromixer. In this embodiment, the substrate diketene experienced a complete conversion, and the target product L-carnitine had a total yield of 63% and a purity of 99%.

### Example 3

**Example 3** was basically the same as the **Example 1,** except that the microchannel reactor used herein for chlorination reaction was a tubular microchannel reactor with a volume of 10 mL and an inner diameter of 0.8 mm. In this embodiment, the substrate diketene experienced a complete conversion, and the target product L-carnitine had a total yield of 60% and a purity of 99%.

### Example 4

**Example 4** was basically the same as the **Example 1,** except that the back pressure adopted herein was 0.5 MPa. In this embodiment, the substrate diketene experienced a complete conversion, and the target product L-carnitine had a total yield of 60% and a purity of 99%.

### Example 5

**Example 5** was basically the same as the **Example 1,** except that the molar ratio of diketene to chlorine gas was 1:1.3. In this embodiment, the substrate diketene experienced a complete conversion, and the target product L-carnitine had a total yield of 55% and a purity of 99%.

### Example 6

**Example 6** was basically the same as the **Example 1,** except that the molar ratio of diketene to ethanol was 1:1.2. In this embodiment, the substrate diketene experienced a complete conversion, and the target product L-carnitine had a total yield of 63% and a purity of 99%.

### Example 7

**Example 7** was basically the same as the **Example 1,** except that the molar ratio of diketene to trimethylamine was 1:1.5. In this embodiment, the substrate diketene experienced a complete conversion, and the target product L-carnitine had a total yield of 65% and a purity of 99%.

It should be noted that the embodiments are merely illustrative, and are not intended to limit the scope of the application. It should be understood that any changes, improvements and modifications made by those skilled in the art without departing from the spirit shall fall within the scope of the present application defined by the appended claims.

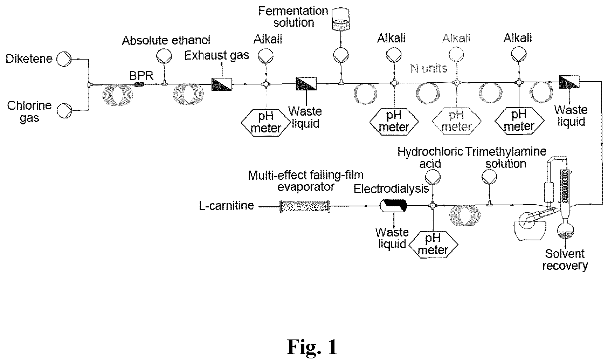

## Claims

1. A full continuous-flow preparation method of L-carnitine using a micro-reaction system, the micro-reaction system comprising a first micromixer, a first microchannel reactor, a second micromixer, a second microchannel reactor, a third micromixer, a third microchannel reactor, a fourth micromixer and a fourth microchannel reactor communicated in sequence; and the method comprising:
(S1) respectively transporting chlorine gas and a diketene reaction liquid to the first micromixer for mixing, and allowing the reaction mixture in the first micromixer to flow into the first microchannel reactor followed by continuous chlorination reaction;
(S2) transporting the reaction mixture flowing out from the first microchannel reactor and an alcohol solvent into the second micromixer and the second microchannel reactor in sequence for continuous esterification reaction;
(S3) neutralizing the reaction mixture flowing out from the second microchannel reactor with a first alkali followed by continuous extraction and separation to collect a first organic phase; simultaneously transporting the first organic phase and an aqueous solution of a reductase to a third micromixer and a third microchannel reactor for continuous reduction reaction;
(S4) removing the reductase from the reaction mixture flowing out from the third microchannel reactor via an extraction separator followed by continuous extraction and separation to obtain a second organic phase; and concentrating the second organic phase to obtain a concentrated liquid;
(S5) simultaneously transporting the concentrated liquid obtained in step (S4) and a trimethylamine solution to a fourth micromixer and a fourth microchannel reactor in sequence for continuous substitution and hydrolysis reaction; and
(S6) neutralizing the reaction mixture flowing out from the fourth microchannel reactor with a second alkali followed by feeding into a demineralization device and a concentration device to obtain the L-carnitine.

2. The full continuous-flow preparation method according to claim 1, **characterized in that** the diketene reaction liquid is a solution of diketene in an organic solvent; the organic solvent is selected from the group consisting of benzene, methylbenzene, an ester solvent, a chlorinated hydrocarbon solvent and an alkane solvent;
the reductase is a carbonyl reductase; and
the trimethylamine solution is a trimethylamine aqueous solution or a trimethylamine alcoholic solution.

3. The full continuous-flow preparation method according to claim 1, **characterized in that** in step (S1), a flow ratio of the chlorine gas to the diketene reaction liquid is controlled such that a molar ratio of diketene to the chlorine gas is 1:(0.9~1.5); the first micromixer and the first microchannel reactor are both controlled at -20~50°C; a residence time of the reaction mixture in the first microchannel reactor is 0.1~30 min; and a back pressure during the continuous chlorination reaction is controlled to 0.1~1.0 MPa.

4. The full continuous-flow preparation method according to claim 1, **characterized in that** in step (S2), the alcohol solvent is ethanol; a flow ratio of the reaction mixture obtained in step (S1) to ethanol is controlled such that a molar ratio of diketene to ethanol is 1:(0.9~1.5); the second micromixer and the second microchannel reactor are both controlled at 20-80°C; a residence time of the reaction mixture in the second microchannel reactor is 0.1-30 min; and a back pressure during the continuous esterification reaction is controlled to 0.1-1.0 MPa.

5. The full continuous-flow preparation method according to claim 1, **characterized in that** in step (S3), the third micromixer and the third microchannel reactor are both controlled at 20-50°C; a residence time of the reaction mixture in the third microchannel reactor is 1-30 min; and a back pressure during the continuous reduction reaction is controlled to 0.1-1.0 MPa.

6. The full continuous-flow preparation method according to claim 1, **characterized in that** in step (S5), a flow ratio of the concentrated liquid obtained in step (S4) to the trimethylamine solution is controlled such that a molar ratio of diketene to trimethylamine is 1:(1.0~2.0); the fourth micromixer and the fourth microchannel reactor are both controlled at 20-50°C; a residence time of the reaction mixture in the fourth microchannel reactor is 1-30 min; and a back pressure during the continuous substitution and hydrolysis reaction is 0.1-1.0 MPa.

7. The full continuous-flow preparation method according to claim 1, **characterized in that** the first micromixer, the second micromixer, the third micromixer and the fourth micromixer are each independently selected from the group consisting of static mixer, T-type micromixer, Y-type micromixer, cross-type mixer, coaxial flow micromixer and flow-focusing micromixer; and the first microchannel reactor, the second microchannel reactor, the third microchannel reactor and the fourth microchannel reactor are each independently a tubular microchannel reactor, a plate-type microchannel reactor, or other commercially available microchannel reactors.

8. The full continuous-flow preparation method according to claim 7, **characterized in that** an inner diameter of the tubular microchannel reactor is 50 µm-10 mm; the plate-type microchannel reactor comprises a first heat exchange layer, a reaction layer and a second heat exchange layer arranged in sequence from top to bottom; the reaction layer is provided with a reaction fluid channel; and a hydraulic diameter of the reaction fluid channel is 50 µm-10 mm.

9. The full continuous-flow preparation method according to claim 1, **characterized in that** the continuous extraction separator is a plate-type microchannel extraction separator, a membrane extraction separator, an annular centrifugal extraction separator, a ceramic membrane separator, an electrodialysis device, a continuous concentration device, or a multi-effect falling-film evaporator.

## Patentansprüche

1. Vollständiges kontinuierliches Durchflussverfahren zur Herstellung von L-Carnitin unter Verwendung eines Mikroreaktionssystems, wobei das Mikroreaktionssystem einen ersten Mikromischer, einen ersten Mikrokanalreaktor, einen zweiten Mikromischer, einen zweiten Mikrokanalreaktor, einen dritten Mikromischer, einen dritten Mikrokanalreaktor, einen vierten Mikromischer und einen vierten Mikrokanalreaktor umfasst, die in Reihe geschaltet sind; und das Verfahren Folgendes umfasst:
(S1) Getrenntes Zuführen von Chlorgas und einer Diketen-Reaktionsflüssigkeit zum ersten Mikromischer zum Mischen und Ermöglichen des Einströmens des Reaktionsgemisches aus dem ersten Mikromischer in den ersten Mikrokanalreaktor, gefolgt von einer kontinuierlichen Chlorierungsreaktion;
(S2) Zuführen des aus dem ersten Mikrokanalreaktor ausfließenden Reaktionsgemisches und eines Alkohol-Lösungsmittels nacheinander in den zweiten Mikromischer und den zweiten Mikrokanalreaktor für eine kontinuierliche Veresterungsreaktion;
(S3) Neutralisieren des aus dem zweiten Mikrokanalreaktor ausfließenden Reaktionsgemisches mit einer ersten Lauge, gefolgt von einer kontinuierlichen Extraktion und Trennung zur Sammlung einer ersten organischen Phase; gleichzeitiges Zuführen der ersten organischen Phase und einer wässrigen Lösung einer Reduktase zu einem dritten Mikromischer und einem dritten Mikrokanalreaktor für eine kontinuierliche Reduktionsreaktion;
(S4) Entfernen der Reduktase aus dem dritten Mikrokanalreaktor ausfließenden Reaktionsgemisch mittels eines Extraktionsseparators, gefolgt von einer kontinuierlichen Extraktion und Trennung zur Gewinnung einer zweiten organischen Phase; und Konzentrieren der zweiten organischen Phase zur Gewinnung einer konzentrierten Flüssigkeit;
(S5) gleichzeitiges Zuführen der in Schritt (S4) erhaltenen konzentrierten Flüssigkeit und einer Trimethylaminlösung zu einem vierten Mikromischer und einem vierten Mikrokanalreaktor nacheinander für eine kontinuierliche Substitutions- und Hydrolysereaktion; und
(S6) Neutralisieren des aus dem vierten Mikrokanalreaktor ausfließenden Reaktionsgemisches mit einer zweiten Lauge, gefolgt von der Zufuhr in eine Entmineralisierungsvorrichtung und eine Konzentrationsvorrichtung zur Gewinnung von L-Carnitin.

2. Vollständiges kontinuierliches Durchflussverfahren zur Herstellung nach Anspruch 1, **dadurch gekennzeichnet, dass** die Diketen-Reaktionsflüssigkeit eine Lösung von Diketen in einem organischen Lösungsmittel ist; das organische Lösungsmittel aus der Gruppe ausgewählt ist, die aus Benzol, Methylbenzol, einem Esterlösungsmittel, einem chlorierten Kohlenwasserstofflösungsmittel und einem Alkanlösungsmittel besteht;
die Reduktase eine Carbonylreduktase ist; und
die Trimethylaminlösung eine wässrige Trimethylaminlösung oder eine alkoholische Trimethylaminlösung ist.

3. Vollständiges kontinuierliches Durchflussverfahren zur Herstellung nach Anspruch 1, **dadurch gekennzeichnet, dass** in Schritt (S1) ein Strömungsverhältnis des Chlorgases zur Diketen-Reaktionsflüssigkeit so gesteuert wird, dass ein Molverhältnis von Diketen zu Chlorgas 1:(0,9-1,5) beträgt; der erste Mikromischer und der erste Mikrokanalreaktor beide auf -20 bis 50 °C geregelt werden; die Verweilzeit des Reaktionsgemisches im ersten Mikrokanalreaktor 0,1 bis 30 Minuten beträgt; und der Gegendruck während der kontinuierlichen Chlorierungsreaktion auf 0,1 bis 1,0 MPa geregelt wird.

4. Vollständiges kontinuierliches Durchflussverfahren zur Herstellung nach Anspruch 1, **dadurch gekennzeichnet, dass** in Schritt (S2) das alkoholische Lösungsmittel Ethanol ist; das Strömungsverhältnis des in Schritt (S1) erhaltenen Reaktionsgemisches zu Ethanol so gesteuert wird, dass ein Molverhältnis von Diketen zu Ethanol 1:(0,9-1,5) beträgt; der zweite Mikromischer und der zweite Mikrokanalreaktor beide auf 20 bis 80 °C geregelt werden; die Verweilzeit des Reaktionsgemisches im zweiten Mikrokanalreaktor 0,1 bis 30 Minuten beträgt; und der Gegendruck während der kontinuierlichen Veresterungsreaktion auf 0,1 bis 1,0 MPa geregelt wird.

5. Vollständiges kontinuierliches Durchflussverfahren zur Herstellung nach Anspruch 1, **dadurch gekennzeichnet, dass** in Schritt (S3) der dritte Mikromischer und der dritte Mikrokanalreaktor beide auf 20 bis 50 °C geregelt werden; die Verweilzeit des Reaktionsgemisches im dritten Mikrokanalreaktor 1 bis 30 Minuten beträgt; und der Gegendruck während der kontinuierlichen Reduktionsreaktion auf 0,1 bis 1,0 MPa geregelt wird.

6. Vollständiges kontinuierliches Durchflussverfahren zur Herstellung nach Anspruch 1, **dadurch gekennzeichnet, dass** in Schritt (S5) das Strömungsverhältnis der in Schritt (S4) erhaltenen konzentrierten Flüssigkeit zur Trimethylaminlösung so gesteuert wird, dass ein Molverhältnis von Diketen zu Trimethylamin 1:(1,0-2,0) beträgt; der vierte Mikromischer und der vierte Mikrokanalreaktor beide auf 20 bis 50 °C geregelt werden; die Verweilzeit des Reaktionsgemisches im vierten Mikrokanalreaktor 1 bis 30 Minuten beträgt; und der Gegendruck während der kontinuierlichen Substitutions- und Hydrolysereaktion 0,1 bis 1,0 MPa beträgt.

7. Vollständiges kontinuierliches Durchflussverfahren zur Herstellung nach Anspruch 1, **dadurch gekennzeichnet, dass** der erste Mikromischer, der zweite Mikromischer, der dritte Mikromischer und der vierte Mikromischer jeweils unabhängig voneinander aus der Gruppe ausgewählt sind, die aus statischem Mischer, T-förmigem Mikromischer, Y-förmigem Mikromischer, Kreuzmischer, koaxialem Durchfluss-Mikromischer und durchflussfokussierendem Mikromischer besteht; und der erste Mikrokanalreaktor, der zweite Mikrokanalreaktor, der dritte Mikrokanalreaktor und der vierte Mikrokanalreaktor jeweils unabhängig voneinander ein röhrenförmiger Mikrokanalreaktor, ein plattenförmiger Mikrokanalreaktor oder andere handelsübliche Mikrokanalreaktoren sind.

8. Vollständiges kontinuierliches Durchflussverfahren zur Herstellung nach Anspruch 7, **dadurch gekennzeichnet, dass** der Innendurchmesser des röhrenförmigen Mikrokanalreaktors 50 µm bis 10 mm beträgt; der plattenförmige Mikrokanalreaktor eine erste Wärmeaustauschschicht, eine Reaktionsschicht und eine zweite Wärmeaustauschschicht umfasst, die von oben nach unten nacheinander angeordnet sind; die Reaktionsschicht mit einem Reaktionsfluidkanal versehen ist; und der hydraulische Durchmesser des Reaktionsfluidkanals 50 µm bis 10 mm beträgt.

9. Vollständiges kontinuierliches Durchflussverfahren zur Herstellung nach Anspruch 1, **dadurch gekennzeichnet, dass** der kontinuierliche Extraktionsseparator ein plattenförmiger Mikrokanal-Extraktionsseparator, ein Membran-Extraktionsseparator, ein ringförmiger Zentrifugal-Extraktionsseparator, ein Keramikmembran-Separator, eine Elektrodialysevorrichtung, eine kontinuierliche Konzentrationsvorrichtung oder ein Mehrfacheffekt-Fallfilmverdampfer ist.

## Revendications

1. Procédé de préparation en flux continu complet de L-carnitine utilisant un système de micro-réaction, le système de micro-réaction comprenant un premier micromélangeur, un premier réacteur microcanalique, un deuxième micromélangeur, un deuxième réacteur microcanalique, un troisième micromélangeur, un troisième réacteur microcanalique, un quatrième micromélangeur et un quatrième réacteur microcanalique communiqués en séquence ; et la méthode comprenant :
(S1) respectivement transporter le gaz chlorure et un liquide de réaction de dikétène vers le premier micromélangeur pour le mélange, et permettre au mélange réactionnel dans le premier micromélangeur de s'écouler dans le premier réacteur microcanalique suivi d'une réaction de chloration continue ;
(S2) transporter le mélange réactionnel sortant du premier réacteur microcanalique et un solvant alcoolique vers le deuxième micromélangeur et le deuxième réacteur microcanalique en séquence pour une réaction d'esterification continue ;
(S3) neutraliser le mélange réactionnel sortant du deuxième réacteur microcanalique avec un premier alcali suivi d'une extraction et d'une séparation continues pour recueillir une première phase organique ; transporter simultanément la première phase organique et une solution aqueuse d'une réductase vers un troisième micromélangeur et un troisième réacteur microcanalique pour une réaction de réduction continue ;
(S4) retirer la réductase du mélange réactionnel sortant du troisième réacteur microcanalique via un séparateur d'extraction suivi d'une extraction et d'une séparation continues pour obtenir une deuxième phase organique ; et concentrer la deuxième phase organique pour obtenir un liquide concentré ;
(S5) transporter simultanément le liquide concentré obtenu à l'étape (S4) et une solution de triméthylamine vers un quatrième micromélangeur et un quatrième réacteur microcanalique en séquence pour une réaction de substitution et d'hydrolyse continue ; et
(S6) neutraliser le mélange réactionnel sortant du quatrième réacteur microcanalique avec un deuxième alcali suivi de l'alimentation dans un dispositif de déminéralisation et un dispositif de concentration pour obtenir la L-carnitine.

2. Procédé de préparation en flux continu complet selon la revendication 1, **caractérisé en ce que** le liquide de réaction du dikétène est une solution de dikétène dans un solvant organique ; le solvant organique est choisi parmi le groupe constitué du benzène, du méthylbenzène, d'un solvant ester, d'un solvant hydrocarboné chloré et d'un solvant alcane ;
la réductase est une réductase de carbonyle ; et
la solution de triméthylamine est une solution aqueuse de triméthylamine ou une solution alcoolique de triméthylamine.

3. Procédé de préparation en flux continu complet selon la revendication 1, **caractérisé en ce qu'**à l'étape (S1), le rapport de débit du gaz chlorure par rapport au liquide de réaction de dikéthène est contrôlé de sorte qu'un rapport molaire de dikéthène par rapport au gaz chlorure soit de 1:(0,9-1,5) ; le premier micromélangeur et le premier réacteur microcanalique sont tous deux contrôlés à -20~50°C ; un temps de séjour du mélange réactionnel dans le premier réacteur microcanalique est de 0,1 à 30 min ; et une pression de retour pendant la réaction de chloration continue est contrôlée à 0,1~1,0 MPa.

4. Procédé de préparation en flux continu complet selon la revendication 1, **caractérisé en ce qu'**à l'étape (S2), le solvant alcoolique est l'éthanol ; un rapport de débit du mélange réactionnel obtenu à l'étape (S1) à l'éthanol est contrôlé de telle sorte qu'un rapport molaire du dikétène à l'éthanol soit de 1:(0,9-1,5) ; le deuxième micromélangeur et le deuxième réacteur microcanalique sont tous les deux contrôlés à 20-80°C ; un temps de séjour du mélange réactionnel dans le deuxième réacteur microcanalique est de 0,1 à 30 min ; et une pression de retour pendant la réaction d'estérification continue est contrôlée à 0,1-1,0 MPa.

5. Procédé de préparation en flux continu complet selon la revendication 1, **caractérisé en ce qu'**à l'étape (S3), le troisième micromélangeur et le troisième réacteur microcanalique sont tous deux contrôlés à 20-50°C ; un temps de séjour du mélange réactionnel dans le troisième réacteur microcanalique est de 1 à 30 min ; et une pression de retour pendant la réaction de réduction continue est contrôlée à 0,1-1,0 MPa.

6. Procédé de préparation en flux continu complet selon la revendication 1, **caractérisé en ce qu'**à l'étape (S5), un rapport de débit du liquide concentré obtenu à l'étape (S4) à la solution de triméthylamine est contrôlé de telle sorte qu'un rapport molaire de dikétène à triméthylamine soit de 1:(1,0-2,0) ; le quatrième micromélangeur et le quatrième réacteur microcanalique sont tous deux contrôlés à 20-50°C ; un temps de séjour du mélange réactionnel dans le quatrième réacteur microcanalique est de 1 à 30 min ; et une pression de retour pendant la réaction de substitution et d'hydrolyse continue est de 0,1 à 1,0 MPa.

7. Procédé de préparation en flux continu complet selon la revendication 1, **caractérisé en ce que** le premier micromélangeur, le deuxième micromélangeur, le troisième micromélangeur et le quatrième micromélangeur sont chacun sélectionnés indépendamment parmi le groupe constitué de mélangeur statique, micromélangeur de type T, micromélangeur de type Y, mélangeur de type croisé, micromélangeur à flux coaxial et micromélangeur à focalisation de flux ; et le premier réacteur microcanalique, le deuxième réacteur microcanalique, le troisième réacteur microcanalique et le quatrième réacteur microcanalique sont chacun indépendamment un réacteur microcanalique tubulaire, un réacteur microcanalique de type plaque, ou autre réacteurs microcanaliques disponibles commercialement.

8. Procédé de préparation en flux continu complet selon la revendication 7, **caractérisé en ce qu'**un diamètre intérieur du réacteur microcanalique tubulaire est de 50 µm-10 mm ; le réacteur microcanalique de type plaque comprend une première couche d'échange thermique, une couche de réaction et une deuxième couche d'échange thermique disposées en séquence de haut en bas ; la couche de réaction est pourvue d'un canal de fluide de réaction ; et un diamètre hydraulique du canal de fluide de réaction est de 50 µm à 10 mm.

9. Procédé de préparation en flux continu complet selon la revendication 1, **caractérisé en ce que** le séparateur d'extraction continue est un séparateur d'extraction à microcanal de type plaque, un séparateur d'extraction à membrane, un séparateur d'extraction centrifuge annulaire, un séparateur à membrane céramique, un dispositif d'électrodialyse, un dispositif de concentration continue ou un évaporateur à film de chute multi-effets.
